# EUROPEAN PATENT APPLICATION

(11) **EP 2 740 404 A1**
(43) Date of publication of application: **11.06.2014**
(21) Application number: 12195569.4
(22) Date of filing: 04.12.2012
(51) Int. Cl.: A61B 5/22, A61B 5/03

(54) **An intraoral interface pressure monitoring system**

(71) Applicant: University of Limerick, Co. Limerick Limerick (IE)
(72) Inventor: Casey, Vincent, Bruff, Co. Limerick (IE); Conway, Richard, Annacotty, Co. Limerick (IE); Perry, Alison, Killaloe, Co. Clare (IE)
(74) Representative: Purdylucey Intellectual Property

(57) **Abstract**

An intraoral interface pressure measurement system which is useful for estimating the pressure and pressure transients produced by oral tissues. The intraoral interface pressure measurement system comprises a modified MEMS pressure sensor (21) in a conformable semisolid body (20) which measures the pressure applied by the selected tissue to other surfaces within the oral cavity, and communicates this and other data to a control means (3) which in turn wirelessly communicates the data to a remote data logger (11).

## Description

### FIELD OF THE INVENTION

This invention pertains to an intraoral pressure monitoring system for safely estimating the pressure at intraoral tissues interfaces in order to improve assessment, diagnosis and clinical therapeutic outcomes. The invention also relates to a method of measuring intraoral pressure at a tissue interface within the oral cavity, which method employs an intraoral interface pressure transducer apparatus

### BACKGROUND OF THE INVENTION

The tongue is an important organ for clear articulation of alveolo-lingual (t, d) and velar (k, g) plosive speech sounds. The ability to generate a pressure between the tongue tip and alveolar ridge or between the posterior tongue and velum are important predictors of speech clarity in both children and adults. In people with a dysarthria (a motor speech disorder), such pressures are often low or absent.

The tongue is also essential during both the oral and the pharyngeal phases of swallowing, being primarily responsible for bolus propulsion during deglutition. The tongue comprises muscle, connective, and adipose tissues and is covered in both keratinised and non-keratinised squamous cell epithelium. For descriptive purposes, it is commonly divided into three primary sections; the anterior, medial, and posterior tongue. Significant differences in the levels of muscle, connective, and adipose tissue are found across these three sections of the tongue. These differences exist as a result of the different functions that each of these areas contribute to speech and swallowing. Upon initiation of the swallow, the anterior and medial sections of the tongue maximally elevate in a sequential manner to form a firm lingua-palatal contact area that sweeps posteriorly, forcing the bolus into the pharynx. While the tongue works as a whole to propel the bolus into the pharynx, the nature of the movement and the magnitude, duration, and timing of the lingua-palatal contact made by each section of the tongue during swallowing, are distinctly different.

Both head and neck (H&N) cancer and its treatments have been shown to significantly affect tongue function during swallowing thereby impairing deglutitive tongue function. As a result, patients often display significantly increased oral transit times and increased levels of post-swallow oral residue, when compared with normal controls, which puts them at risk of aspiration (choking) and resulting chest infections/pneumonia. Dysphagia (a swallowing disorder) may also arise after a stroke or other neurogenic condition, and in the elderly. In rehabilitation of dysphagia arising from any aetiology it is desirable for clinicians to have a reliable method to measure (isotonic and swallowing) tongue function within the patient population. It is also necessary to have an accurate measurement of deglutitive tongue function in order to have a baseline against which to examine changes to function after therapy and/or surgical intervention. Oro-lingual swallowing pressures are used as a measure of deglutitive tongue function - i.e., a surrogate measure of swallowing efficiency.

Physiological information such as tongue pressure must be obtained in a standardized fashion to assess the strength of tongue placement during speech production and the peristaltic function of the tongue in the swallowing process. The pressure at various interfaces such as between the tongue and specific sites on the palate, gums, teeth and cheeks may also be of clinical interest. Similarly the pressure at the interface between the lips may have clinical utility for the assessment and treatment of certain pathologies.

Differing technologies and methods have been used to capture pressures at various oral tissue interfaces. There are various known types of accurate high performance commercially available pressure sensor device which may be incorporated into biomedical interface pressure measurement systems. For instance there are known pressure sensor devices that are operated pneumatically, electrically, piezoelectrically, piezoresistively or using strain gauges. Such pressure sensors are typically optimised for hydrostatic pressure measurement and so have optimum performance when the target pressure is relayed via fluid such as gas or liquid to the primary transduction element which is normally a cantilever or diaphragm.

With water-perfused intraoral pressure measurement systems, a pneumohydraulic pump perfuses distilled water through 3-8 lumens of the multilumen manometric catheter, each of which is connected to an external volume displacement pressure transducer. Each lumen of the masnometric catheter terminates at a side-hole or sleeve channel within the tongue-palate interface and senses intraluminal pressure at that position by the relative obstruction to flow of the perfusate. Accurate recordings of tongue pressure waves can be made at infusion rates of less than 0.5 ml/min per channel. However, perfused manometric systems require specialist equipment maintenance and operation in order to achieve published response characteristics.

An alternative system uses strain gauge sensors and solid-state electronics components. In these manometric systems, the manometric probe contains the transducers (strain gauges) at fixed locations along its length. The probe plugs into a small box containing the electronics, which may then be connected to a recorder or data acquisition system. These systems have excellent response times (typically 0-20,000 Hz) and are less cumbersome than the perfusion pumps. The main disadvantages of the solid state strain gauge systems are: the manometric probes are expensive particularly for multichannel versions; are sometimes fragile and unmodifiable; and, are subject to several physical constraints with respect to the number of sensing elements and the proximity of the elements to each other.

Yet another system, known as the sleeve sensor, which typically contains a 6-cm long silicone membrane under which water is perfused is used for oral and oesophageal pressure measurement. When pressure is applied anywhere along the length of the membrane, the resistance to water flow beneath that membrane increases and pressure registers on that manometric channel. This will be the highest pressure acting anywhere along its length. The system requires three additional channels to 'mark' the start, middle and end of the sleeve itself and it has very limited frequency response making it unsuitable for the measurement of fast pressure wave rise and fall times.

A number of systems used in oro-lingual pressure measurement deploy a gas filled bubble or capsule as the intraoral probe. Compression of the capsule produces a pressure increase in the trapped gas which is relayed to a pressure transducer, remote from the bubble, using a gas filled line. Two commercial systems based on this principle are the Iowa Oral Performance Instrument (IOPI) and the Kay Swallowing Workstation's (KSW) three-transducer tongue pressure array. While these systems are used clinically, there is considerable variability in the data which may arise because of artefacts associated with the intrusiveness of the probes used. Additionally, data reliability requires sophisticated, carefully controlled evaluations obtained using relatively high level, often customized instrumentation and clear study protocols, tailored to answer specific physiological questions on a case-by-case basis or to compare the physiological responses among groups of individuals. This usage is generally reserved for research settings in specialized centres. When used in clinical settings, data unreliability severely limits the utility of such measurements for pathological and rehabilitation assessment.

Accordingly, there is a need for a minimally intrusive intraoral interface pressure monitoring system which provides a reliable, time resolved estimate of the actual pressure applied by tissue such as the tongue, cheeks and lips, to other structures within the oral cavity, in a continuous dynamic way. The availability of such a system would greatly enhance the clinical utility of, for instance, oro-lingual pressure measurement in the diagnosis and treatment of both dysarthria and dysphagia through improved data reliability and simpler use protocols requiring lower operator skill levels than current systems.

Of the various electrical device properties which may be exploited in pressure sensors, electrical resistance is the easiest one to measure precisely over a wide range at moderate cost. Low profile, small footprint microelectromechanical system (MEMS) pressure sensor devices are widely available for general purpose, low cost, pressure measurement applications, such as watch altimeters and diving depth gauges. The small size of these devices makes them attractive candidate pressure sensors for inclusion in improved biomedical interface pressure transducers designed to eliminate the need for fluid lines by locating the sensor directly at the interface measurement site. These devices are typically piezoresistive, transforming a mechanical stress into a resistance signal which may be further transformed, through suitable electronic processing, into a pressure signal. However, as with the conventional pressure sensors mentioned above, they are also typically optimized for hydrostatic pressure measurement and will not perform to specification if coupled directly to continuum media such as membranes or tissue. However, known so-called media isolation MEMS pressure sensors have been developed recently for specialist medical applications which can be operated while in direct contact with viscoelastic continuum media such as tubing and polymer membranes. However, these devices are optimized for switching applications rather than continuous pressure monitoring use.

The applicant is aware of the following published references which are more or less relevant to the subject matter of the applicant's invention:
R White, S. M. Cotton, J. Hind, J. Robbins and A. Perry, "A comparison of the reliability and stability of oro-lingual swallowing pressures in patients with head and neck cancer and healthy adults.", Dysphagia, 2009, 24: 137-144.
V. Casey, S. Griffin and S. B. G. O'Brien, "An investigation of the hammocking effect associated with interface pressure measurements using compression bandage cuffs", Medical Engineering & Physics, V23, 2001, pp. 513-519;
S. B. G. O'Brien and V. Casey, "Asymptotic and numerical solutions for a hammocking model", Quarterly Journal of Mechanics and Applied Mathematics, V55, 2002, pp. 409-420;
Casey, V, McAree, B., Grace, P, Clarke Moloney, M, "Wearable Sub-bandage Pressure Measurement System", IEEE SENSORS APPLICATIONS SYMPOSIUM SAS 2010, Limerick.
McEwen, J A, Casey V, " Measurement of hazardous pressure levels and gradients produced on human limbs by non-pneumatic tourniquets", CMBEC32, Vancouver, 2009.
Casey, V, Little, E., "Novel interface pressure transducers for tourniquets and other medical devices.2, CMBEC33, Vancouver, 2010.
Intersema Application Note AN401, entitled Analog Sensor Interfacing.
P. J. Kahrilas, R. E. Clouse and W. J. Hogan, "American Gastroenterological Association technical review on the clinical use of esophageal manometry." Gastroenterology, 1994, 107: 1865-1884.
Telegesis Zigbee modules, http://www.telegesis.com/default.htm
Zigbee Alliance home page, http://www.zigbee.org/
Zigbee Alliance, "Zigbee Wireless Sensor Applications for Health, Wellness and Fitness", March, 2009, https://docs.zigbee.org/zigbee-docs/dcn/09-4962.pdf

It is an object of the invention to overcome at least one of the above-referenced problems.

### SUMMARY OF THE INVENTION

Accordingly, this invention is directed towards providing an improved intraoral interface pressure measurement system for safely estimating the pressures arising at tissue interfaces within the mouth in order to improve diagnosis and the efficacy of clinical therapeutic outcomes. The system uses a novel biomedical interface pressure transducer which incorporates low cost, un-calibrated or factory pre-calibrated, MEMS pressure sensors, specially adapted or modified to be located within the oral cavity at preselected pressure measurement sites.

In a first aspect, the invention relates to a method of measuring intraoral pressure at a tissue interface within the oral cavity, which method employs an intraoral interface pressure transducer apparatus comprising (a) a pressure transducer and (b) control means in communication with the pressure transducer and configured to provide interfacing, data storage and/or display functions. Typically, the pressure transducer comprises a conformable semi-solid body that in use is capable of at least partially conforming to the shape of a supporting tissue surface in the oral cavity, and a modified MEMS pressure sensor contained within the conformable semi-solid body, the modified MEMS pressure sensor having a pressure sensor port adapted to allow direct contact to continuum materials and configured to generate a pressure signal indicative of interface pressure applied to the tissue within the oral cavity. The method generally comprises the step of locating the intraoral interface pressure transducer in the oral cavity at a supporting tissue site, wherein the modified MEMS pressure sensor produces a pressure signal indicative of the intraoral pressure generated by the tongue at that site which is communicated to the control means.

The invention also relates to the use of an intraoral interface pressure transducer apparatus for the measurement of intraoral pressure at a tissue interface within the oral cavity, wherein the intraoral interface pressure transducer apparatus comprises (a) a pressure transducer and (b) control means in communication with the pressure transducer and configured to provide interfacing, data storage and display function, the pressure transducer comprising:
- a conformable semi-solid body that in use is capable of at least partially conforming to the shape of a tissue surface in the oral cavity;
- a modified MEMS pressure sensor contained within the conformable semi-solid body, the modified MEMS pressure sensor having a pressure sensor port adapted to allow direct contact to continuum materials and configured to generate a pressure signal indicative of interface pressure applied to the tissue within the oral cavity.

The invention also relates to a use of an intraoral interface pressure transducer for the measurement of intraoral pressure at a tissue interface within the oral cavity, wherein the intraoral interface pressure transducer comprises a conformable semi-solid body that in use is capable of at least partially conforming to the shape of a supporting tissue surface in the oral cavity, and a modified MEMS pressure sensor contained within the conformable semi-solid body, the modified MEMS pressure sensor having a pressure sensor port adapted to allow direct contact to continuum materials and configured to generate a pressure signal indicative of interface pressure applied to the tissue within the oral cavity.

In a preferred embodiment, the pressure transducer comprises means for affixing the pressure transducer to a supporting tissue surface within the oral cavity, for example a palate, an upper palate, a tongue, a cheek, teeth, an alveolar ridge, in which the method includes a step of affixing the pressure transducer to a tissue surface within the oral cavity using the affixing means. Ideally, the method comprises a step of affixing a bottom surface of the pressure transducer to the palate, ideally the upper palate, of the oral cavity. The bottom surface of the pressure transducer may comprise an adhesion promotion layer of polymer such as polyimide or other polar polymer designed to provide a surface to which fixatives and general purpose adhesives readily bond. Bonding of this adhesion promotion layer to the conformable semi-solid body may be conveniently achieved using an intermediate layer typically comprising a metal foil such as copper which is adhesively joined to the adhesion promotion layer. The copper layer, in turn provides a strong bond to the conformable semi-solid body. Alternatively, pre-punched adhesive tape may be used to affix the transducer to the target measurement site. The use of adhesive fixative and tape may be avoided using integral suction cups formed on the bottom surface of the pressure transducer.

Typically, the pressure transducer is adapted to further provide a signal indicative of the temperature at the measurement site local to the transducer. Such a signal is useful in correcting the pressure signal for temperature induced pressure artefacts. However, it also provides a means of distinguishing between a reflexive saliva swallow and a controlled swallow involving a food or drink bolus provided there is a temperature difference between the bolus temperature and the subject's body temperature. Typically, the bolus temperature will be either elevated or depressed relative to body temperature by at least 5°C. Consequently, a temperature fluctuation will occur coincidently with a swallow induced pressure pulse but be absent for a saliva or reflexive swallow. Therefore, the temperature signal provides a useful marker to distinguish between control and reflexive swallows. Thus, the invention also provides a method of distinguishing between a reflexive saliva swallow and a controlled swallow of a food or drink bolus in a subject, in which there is a temperature difference between the bolus temperature and the subjects body temperature, the method comprising measuring intraoral pressure and temperature at a tissue interface within the oral cavity for a period of time, wherein the detection of a pressure pulse with a coincident temperature fluctuation correlates with a reflexive swallow, or wherein the detection of a pressure pulse without a coincident temperature fluctuation correlates with a reflexive swallow. Suitably, the method of measuring intraoral pressure and temperature employs an intraoral interface pressure transducer apparatus according to the invention.

The pressure transducer may further provide a means for reproducibly locating the transducer in the oral cavity. Such means in the form of a graduated length scale printed onto the flexible circuit may be used to reproducibly place the sensor at standard anterior, medial or posterior positions in the oral cavity by using the scale to indicate the transducer distance from anatomical landmarks such as the teeth or the lips.

The invention also relates to an intraoral interface pressure transducer apparatus for measuring intraoral pressure at a tissue interface within the oral cavity comprising (a) a pressure transducer and (b) control means in communication with the pressure transducer and configured to provide interfacing, data storage and/or display functions, the pressure transducer comprising:
- a conformable semi-solid body that in use is capable of at least partially conforming to the shape of a tissue surface in the oral cavity;
- a modified MEMS pressure sensor contained within the conformable semi-solid body, the modified MEMS pressure sensor having a pressure sensor port adapted to allow direct contact to continuum materials and configured to generate a pressure signal indicative of interface pressure applied to the tissue within the oral cavity; and
- optionally, means for affixing the conformable semi-solid body to a surface of the oral cavity.

In one embodiment, the pressure transducer comprises a sensor array comprising a plurality of modified MEMS pressure sensors, wherein said sensor array generates a plurality of pressure signals indicative of interface pressures applied to the tissue within the oral cavity. In this embodiment, the control means preferably is adapted to provide estimates of pressure gradients and pressure pulse velocities at tissue interfaces.

The means for affixing may be, for example, oral fixative creams and tapes, integrated suction cups, pre-punched adhesive tape with release liner or any general purpose fixative approved for oral use.

Ideally, the affixing means is easily removable from both the tissue and the transducer and is typically disposable.

Suitably, affixing means is disposed on a bottom surface of the pressure transducer (the face of the transducer that is located opposite an opening of the pressure sensor port). However, pre-punched adhesive tape such as Medisilk White tape or 3M Tegaderm™ transparent tape may be fixed to the top face of the transducer provided the tape has a punch hole which is larger in diameter by at least 2 mm than the sensor port diameter and is concentric with the sensor port. The tape extends beyond the perimeter of the transducer in order to provide an adhesive surface free to contact the supporting tissue. The adhesive surface of the tape may be suitably coated with a release liner to facilitate easy placement and transducer deployment.

Generally, the pressure sensor port of the modified MEMS pressure sensor has an internal volume which is partially filled with a bottom layer of MEMS protection material and a top layer of media isolation material such that the top surface of said media solution material is substantially coplanar with the top surface of said pressure sensor port.

Preferably, the modified MEMS pressure sensor is electrically connected to the control means using a pattern conductor on a flexible substrate.

Suitably, the ratio of the area of the top surface of said media isolation material to the footprint area of the MEMS pressure sensor substrate of the modified MEMS pressure sensor is greater than 0.3.

Typically, the conformable body has a top planar face and a bottom planar face disposed substantially parallel to each other, said conformable semisolid body tapering inwardly from the bottom planar face to the top planar face, ideally following a convex curved surface, said top planar face of said conformable semisolid body being substantially coplanar with the top surface of the media isolation material of the modified MEMS pressure sensor and said conformable semisolid body substantially enclosing said modified MEMS pressure sensor.

Preferably, the MEMS protection material is a low viscosity, low hardness, incompressible gel and the media isolation material is a soft, non-stick, incompressible rubber or gel.

Typically, the conformable semisolid body is formed from substantially incompressible material.

Suitably, the conformable semisolid body has physical dimensions and flexibility selected so that it conforms to supporting tissue surfaces with radius of curvature greater than 10mm and in use does not displace the tongue or pressure applying tissue more than 4mm from said supporting tissue surfaces.

In one embodiment, the apparatus further comprises a shear relief membrane with an inner surface and an outer surface, which substantially encloses the conformable semisolid body and the modified MEMS pressure sensor. The shear relief membrane is typically formed from a flexible, elastic material that has a low coefficient of friction. Ideally, a lubrication fluid or gel is distributed over a major proportion of the inner surface of the shear relief membrane.

Typically, the apparatus further comprises a head fixture adapted to support the control means. The head fixture may also anchor the transducer and direct the flexible interconnect cable at an angle to the oral cavity such that it does not interfere with normal food/drink consumption and swallowing. Suitably, the head fixture comprises a head-set adapted to attach to the patient's head and an adjustable boom adapted to house and support the control means and provide electrical interconnectivity between the transducer and the control means. Ideally, the head fixture and adjustable boom are connected by means of a pivot joint enabling relative pivoting movement there between. Furthermore, the boom may be configured to allow adaptation of the overall head fixture - boom - transducer configuration to facilitate differing patient physiology.

Suitably, the control means comprises one or more of power supply means, signal conditioning means, and interfacing, data storage and/or display functions. Preferably, the control means comprises a wireless transceiver for sending and/or receiving data wirelessly. Suitably, the control means is adapted to interface with an electronic processing device such as a computer, PDA, smart phone or the like. Ideally, the control means is configured for continuous real time transmission, display or storage of pressure data, data streams, patient details, measurement conditions. Preferably, the control means is configured to process the data received from the pressure transducer and correlate the received data with clinical information such, for example, diagnosis of disease or conditions. In one embodiment, the data received is manipulated to infer diagnostic and therapeutic efficacy indexes such as peak pressure, peak pressure ratios, pressure rise time, pressure fall time, and pressure impulse (integral of pressure over time).

Preferably, the conformable semi-solid body is formed of a substantially incompressible material, for example a polydimethyl siloxane (PDMS) material, for example SYLGARD 184 (Dow Corning) or another relatively incompressible elastomer.

Typically, the conformable semi-solid body is dimensioned so that it generally conforms to the surface of the oral support tissue. In one embodiment, the body has a generally frustoconical shape with top and bottom faces (surfaces) that are generally parallel and ideally comprises sidewalls that curve gently upwardly from the bottom surface towards the top surface.

In one embodiment, the transducer apparatus comprises a plurality of pressure transducers and a control means in communication with the plurality of pressure transducers. This allows the apparatus to provide pressure wave data at a plurality of locations within the oral cavity.

Typically, each pressure transducer comprises means for measuring temperature at the pressure measurement site.

The invention also provides a kit comprising an intraoral interface pressure transducer apparatus according to the invention and a disposable sleeve, ideally a plurality of disposable sleeves, adapted to removably attach to the pressure transducer such that in use it covers the pressure transducer. The sleeve may comprise a thin elastomeric membrane that is adapted to be removed after use. In one embodiment, the sleeve has an elasticated opening to facilitate encapsulation and removal from the transducer.

The invention also provides a kit comprising an intraoral interface pressure transducer apparatus according to the invention and a data logger with a wireless transceiver, wherein the control means of the transducer apparatus is adapted to establish wireless connectivity with the wireless transceiver of the data logger. Typically, the data logger is used to monitor, display, store and manipulate data so as to present it in a human readable fashion.

The invention also provides a kit comprising an intraoral interface pressure transducer apparatus according to the invention and a computer program stored on a storage medium, the computer program comprising program instruction adapted to process pressure data received from the pressure transducer. For example, the program instructions may be adapted to correlate pressure data with diagnostic or prognostic information, or to infer diagnostic and therapeutic efficacy indexes (for example, peak pressure, peak pressure ratios, pressure rise time, pressure fall time, pressure impulse (integral of pressure over time) and pressure wave velocity), or may be adapted to perform feature extraction using wavelet analysis..

The time series obtained from the pressure transducer exhibits a profile that varies from individual to individual. While the peak pressure value is a defining parameter, it is highly variable for an individual and across individuals. In this invention, software code is provided for feature extraction using wavelet analysis. The use of wavelet analysis enables the extraction of parameters that incorporate the energy in time series profile for a swallow over different frequency bands. This allows both qualitative and quantitative analysis. The time series is transformed into a time-frequency space where the dominant features are more easily observed and any variability is quantifiable. A particular aspect of using wavelet analysis is the selection of the mother wavelet, which has a significant impact on extracting useful features. The wavelet scheme may take an adaptive approach by using a set of standard mother wavelets from which one is selected for an individual. The selection is based on the mother wavelet that best approximates the individual's swallow. The time series for swallow pressure pulses is particularly suited to wavelet analysis due to it's non-stationary characteristics, allowing quantifiable features to be obtained.

In a further aspect, there is provided an intraoral interface pressure measurement system for measuring pressures within the mouth comprising:
a modified MEMS pressure sensor with a solid substrate, said pressure sensor having a pressure sensing port with a top surface and an internal volume which is partially filled with a bottom layer of MEMS protection material and a top layer of media isolation material, said isolation material having an input surface adapted to be locally exposed to a pressure, said input surface being coplanar with the top surface of said pressure sensing port;
a conformable semisolid body containing at least one modified MEMS pressure sensor, said body being defined by circular top and bottom planar faces and a tapered side face, said body having a centrally formed cavity extending from the top planar face through to the bottom planar face, said cavity being shaped to receive the modified MEMS pressure sensor such that said top face is coplanar with the input surface of said pressure sensor and said bottom face is substantially parallel with the solid substrate of said pressure sensor and encloses said sensor end of said flexible substrate and said solid substrate, said conformable semisolid body forming gas tight joints with said pressure sensor;
a telemetry module capable of wireless communications;
a head fixture comprising a rotating and flexible boom;
a flexible circuit with a metallic conductor patterned to define a sensor end and an edge connector end, said sensor end being electrically connected to said modified MEMS pressure sensor, said edge connector end being electrically connected and secured to said lock connector of said boom, said flexible circuit being capable of supporting electrical and electronic components;
a remote data logger in wireless communications with the telemetry module, said data logger providing electrical energy, pressure reference, electronic signal processing, control, signal conditioning, interfacing and display functions;

Typically, the telemetry module is disposed on the head fixture.

The invention suitably provides an apparatus that enables the accurate and reproducible measurement of tissue interface pressures and pressure waves in an oral cavity.

The invention typically also provides an intraoral interface pressure measurement system with long term (days/weeks) calibration stability that requires infrequent calibration checking and allows for simple calibration procedures when such calibration is required.

The invention typically also provides an intraoral interface pressure measurement system that conforms to the shape of certain oral hard tissues thereby reducing the risk of discomfort or injury to the patient or subject and also reducing the overall intrusiveness of the device thereby minimizing errors associated with hammocking and tissue connectivity.

The invention also suitably provides an intraoral interface pressure measurement system that may be comfortably fixed to, or worn on a patient's head without lead wires and cables. This has the advantage of reducing movement related artifacts in the indicated pressure signal. Additionally, it allows the patient adopt an optimally comfortable sitting or standing posture in diagnostic testing and rehabilitation. This is particularly important in elderly patients where their head and neck posture may be compromised or in a more general patient cohort where a 'standard' pose is not possible due to injury or postural limitations.

The invention typically also provides an intraoral interface pressure measurement system that may be interfaced to, and programmatically controlled by a Laptop, PC or other electronic instrument thereby facilitating the continuous real time transmission of pressure data from the apparatus, the display of a measurement data stream or data streams, file storage of measurement data including time and date stamps, patient details and measurement conditions, manipulation of such measurement data to infer diagnostic and therapeutic efficacy indexes such as peak pressure, peak pressure ratios, pressure rise time, pressure fall time, pressure impulse (integral of pressure over time), pressure pulse velocity in a safe and reproducible way.

The invention typically provides an intraoral interface pressure measurement system that may be enclosed in a single use biocompatible, non-allergenic material in order to comply with healthcare hygiene, safety and therapeutic guidelines.

The invention typically provides for automatic compensation and correction of oral pressure measurement data for ambient temperature and pressure variations. In addition, real time temperature data at the measurement site can provide a marker for bolus passage where there is a temperature difference between the patient's body temperature and the temperature of the bolus.

The intraoral interface pressure measurement system may be manufactured inexpensively using low cost materials and components such that embodiments of the transducer may be produced for both single use and multiple use applications.

The oral pressure transducer may be autoclaved using conventional sterilization apparatus or using procedures such as the Tristel Trio 50 wipe four stage process.

### Brief Description of the Drawings

The invention will be more clearly understood from the following description of some embodiments thereof, given by way of example only with reference to the accompanying drawings in which:-
Fig. 1 is a schematic diagram of a preferred embodiment.
Fig. 2 is a cross-sectional view of the intraoral interface pressure transducer of the invention showing details of its construction.
Fig. 3 is an exploded view of the modified MEMS pressure sensor cross section of Fig. 2.
Fig. 4 is a schematic diagram of the intraoral interface pressure transducer of the invention deployed within a human oral cavity.
Fig. 5 is a cross-sectional view of the sensor end of the intraoral interface pressure transducer of Fig. 4 showing details of its deployment between the tongue and palate.
Fig. 6 is a plan view of the edge connector end of the intraoral interface pressure transducer of the present invention.
Fig. 7 is a block diagram showing a schematic representation of the major components of the control means.
Fig. 8 shows a dynamic pressure signal curve obtained from the intraoral interface pressure measurement system of this invention with intraoral interface pressure transducer located close to the alveolar ridge during successive swallow pressure waves.
Fig. 9 shows pressure gradient and integrated pressure impulse data algorithmically generated from pressure data obtained with this invention with intraoral interface pressure transducer located close to the alveolar ridge during successive swallow pressure waves.
Fig. 10 shows three pressure pulses in response to sequential swallowing of three 5 ml water boluses where the bolus is at room temperature. The temperature trace shows clear temperature drops as the bolus is administered thereby providing a convenient marker to distinguish a controlled bolus swallow from a saliva or reflexive swallow.
Fig. 11 shows an pre-punched adhesive tape with release liner which may be used to fix the transducer to supporting tissue.
Fig. 12 is a schematic diagram depicting an alternative embodiment of this invention whereby an integrated array of mini-suction cups is provided for adhesion purposes.
Fig. 13 is a schematic diagram depicting an alternative embodiment of this invention whereby an adhesion promotion layer is bonded to the bottom surface of the transducer.
Fig. 14 is a schematic diagram depicting an alternative embodiment of this invention whereby a graphical scale is provided in order to allow reproducible placement of the transducer in the oral cavity relative to landmarks such as teeth.
Fig. 15 is a schematic diagram depicting an alternative three sensor array embodiment of this invention suitable for multipoint intraoral pressure measurement.
Fig. 16 shows a dynamic pressure pulse sequence obtained from the three sensor array intraoral interface pressure measurement system of this invention with intraoral interface pressure transducers located at standard anterior, medial and posterior landmark locations on the mouth roof.

### DETAILED DESCRIPTION OF THE INVENTION

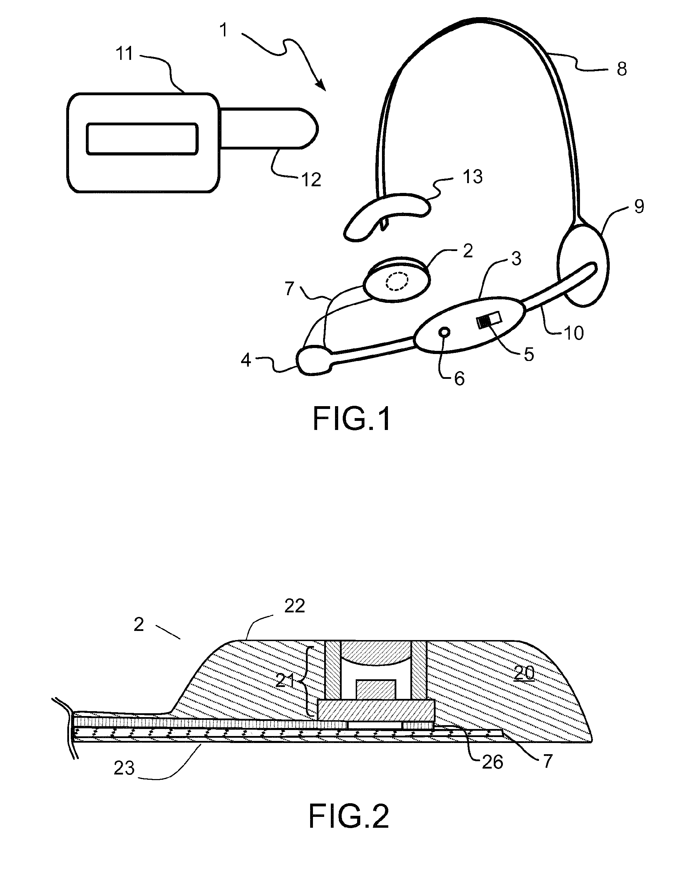

An intraoral interface pressure transducer apparatus 1 is shown in Fig. 1 and consists of: an intraoral interface pressure transducer 2, control means in the form of a telemetry module 3 with on/off switch 5 and power on/data transfer indicator light 6, flexible circuit 7, and head fixture 8 (with rotating pivot 9, adjustable boom 10, cushion 13 and lock interconnect 4). A data logger 11 with USB wireless transceiver 12 adapted to receive data from the telemetry module 3 is also provided.

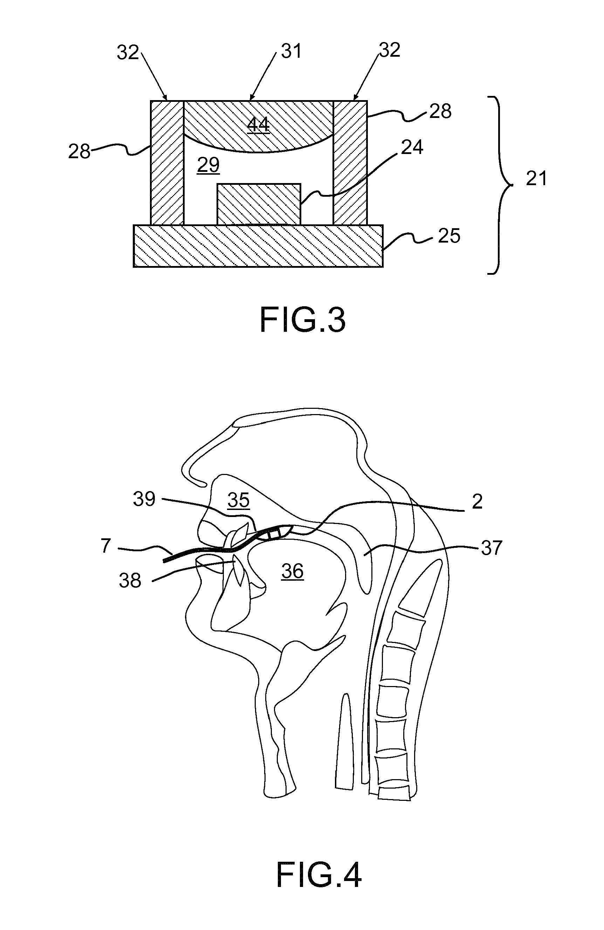

Referring specifically to Figs. 2 and 3, the pressure transducer 2 comprises a conformable semi-solid body 20 of polydimethyl siloxane (PDMS - Dow Corning, Sylgard 184) of thickness less than 4mm and contains a modified MEMS pressure sensor 21. The pressure transducer 2 has generally frustoconical shape, having a top surface 22, a bottom surface 23 that is generally parallel to the top surface 22, and curved sidewalls. The modified MEMS pressure sensor 21 is located centrally within the conformable semi-solid body, and comprises a sensor chip 24 mounted onto a solid substrate 25 which in turn is mounted on a flexible circuit 7 such as Kapton (DuPont) polyamide film with patterned copper conductor tracks 26. The sensor chip 24 is a micromachined peizoresistive pressure sensor, an example of which is manufactured by Measurement Specialities Inc., and is described in the datasheet covering the MS54 series of device.

The thickness extent of the conformable semisolid body 20 is such as to span the vertical extent of the modified MEMS pressure sensor 21 including flexible substrate. As indicated above, in order to minimize hammocking related measurement artefacts, the top face of the conformable semisolid body 22 must be coplanar with the top surface of the MEMS pressure sensor port 32 and the input surface 31 as illustrated in Fig. 2. The opposite face of the conformable semisolid body is substantially parallel to the top face of said body. The conformable semisolid body encapsulates the sensor end of the flexible substrate. However, as such encapsulation adds to the overall thickness of the transducer, it is preferably reduced to small fractions of a millimetre in the region where the flexible substrate supports the modified MEMS pressure sensor as illustrated in Fig. 2. The side walls of the conformable semisolid body are formed in the shape of a convex arc or other such tapered profile in order to allow continuous intimate contact between the intraoral interface pressure transducer and the tissue. An insulating layer covers the conductor tracks in the region between the low profile electrical connector and the conformable semisolid body and may extend some distance into said body (not shown).

Referring specifically to Fig. 3, the modified MEMS pressure sensor 21 comprises a pressure port 28 that is partially filled with MEMS protection material 29 such as soft ion free silicone gel (Dow Corning, Sylgard 527) with viscosity less than 1000cps and low hardness and which has been degassed by being enclosed in a vacuum chamber pumped by a roughing pump for 10 minutes. The MEMS protection material 29 covers the MEMS sensor chip 24 and associated bond wires. A media isolation material 44 fills the remaining volume of the pressure sensor port 28 providing a top surface 31 that is substantially co-planar with a top surface 32 of the pressure port 28. The function of this material is to provide a robust, non-stick interface which may contact continuum materials such as tissue and at the same time translate the pressure applied by the continuum material to the MEMS protection material 29 and MEMS chip 24. The media isolation material 44 may be selected from a range of materials with different Durometer-Shore A hardness index such as RTV Silicone (Dow Corning), polydimethyl siloxane (PDMS) (Dow Corning, Sylgard 184), Silicone Rubber (T20 and T28, Alec Tiranti Limited, Berkshire, UK) and Rubison premixed white silicone. The media isolation material thickness may optimally range from 0.5 mm to 1.5 mm at the point of maximum thickness. Materials with higher Durometer-Shore A index need to be thinner, in general, than softer (low Durometer-Shore A index) materials in order to avoid gel induced sensor response time damping, hysteresis, and sensitivity reduction. The media isolation material must be degassed at 700-750 mmHg vacuum for 20 minutes before use.

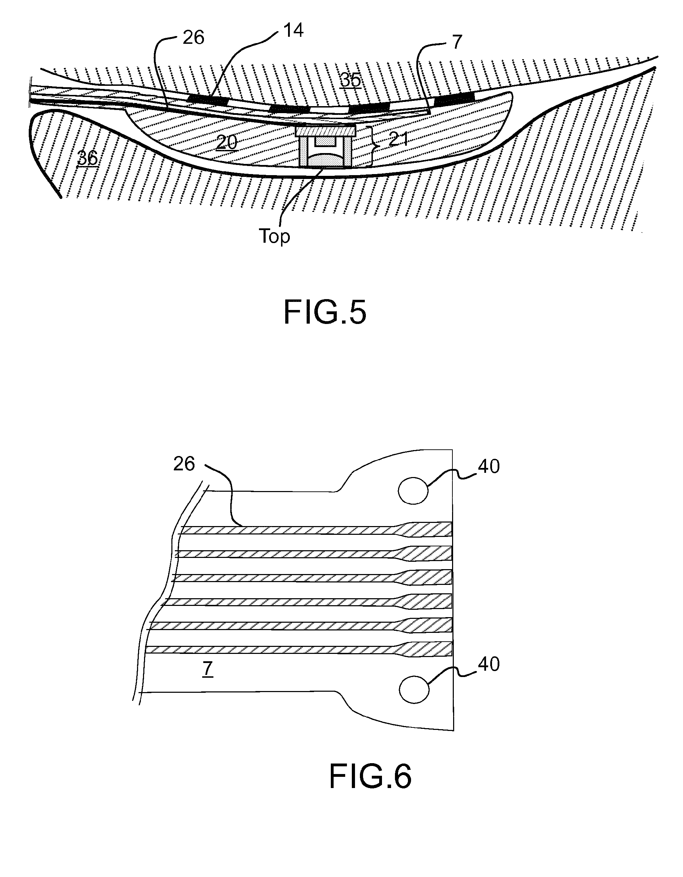

Referring to Figs. 4 and 5, the pressure transducer 2 is shown located within an oral cavity of a user. In this embodiment, the bottom surface 23 of the pressure transducer 2 is affixed to the upper palate 35 of the oral cavity by means of a fixative paste 14 such that the pressure transducer is disposed between the tongue 36 and the upper (hard) palate 35. It will be appreciated that the pressure transducer 2 may be deployed at any one of a number of different locations within the oral cavity, such as between the tongue 36 and the soft palate 37, or between the teeth 38 and the alveolar ridge 39, and may be fixed to the palate using dental fixative creams and pads or may be self-adhering via soft suction cups.

The conformable semisolid body 20 has the general shape of a circular hemispherical section of thickness (height) less than 4 mm. The sides of the conformable semisolid body are tapered between the top face which is orientated towards the pressure applying tissue such as the tongue and bottom face which may be adhesively joined to support tissue such as the palate in such a way as to present a gently contoured transition between the two faces. When tissue such as the tongue applies pressure to the intraoral interface pressure transducer, the conformable semisolid body conforms to the shape of the supporting tissue as illustrated by means of cross-section shown in Fig. 5. Pressure applied by the tongue is transmitted via the shear relief membrane to the input surface of the media isolation material and the modified MEMS pressure sensor. The shear relief membrane (where fitted) has limited freedom to move laterally with respect to the conformable semisolid body, the top surface of the MEMS pressure sensor port and the input surface of the media isolation material within the constraints imposed by the closeness of fit of the membrane to the conformable body. Shear and frictional forces which may exist between the tongue and intraoral interface pressure transducer are substantially reduced by the combined effects of the shear relief membrane and the lubricant coating on the inner surface of said membrane. Since the input surface of the media isolation material is coplanar with the top surface of the sensor port and the top face of the conformable semisolid body, the surface of the tongue and the shear relief membrane will also be substantially coplanar with the input surface of the modified MEMS pressure sensor. Consequently, membrane and tissue lift-off in the vicinity of the input surface of the modified MEMS pressure sensor is minimized or eliminated completely thereby avoiding hammocking related measurement artefacts. Furthermore, saliva and food/drink boluses provide a degree of self lubrication that dissipates shear forces in the vicinity of the input surface. Consequently the input surface is primarily subjected to the normal forces generated by the tongue over the area of the input surface. The sum of such normal forces produces a hydrostatic pressure in the media isolation material and at the MEMS chip that is representative of the pressure applied by the tongue to the support tissue at that location.

Referring to Fig. 6, the interconnect end of the flexible circuit 7 has two punched fixing holes 40 which serve to correctly register the flexible circuit 7 with the fixture connection lock which secures the oral manometer to the fixture assembly.

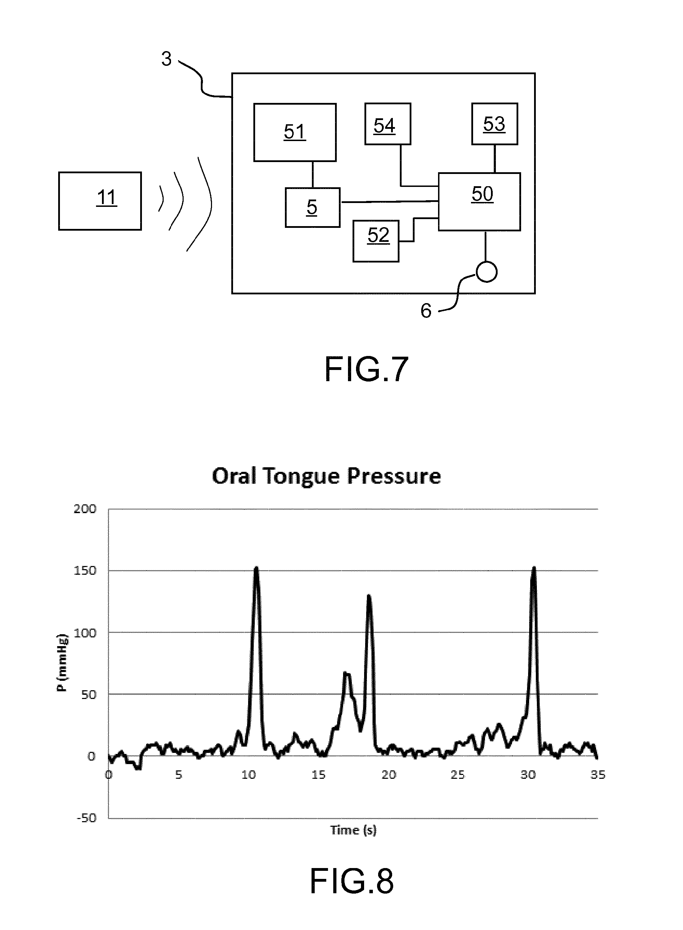

Fig. 7 shows a schematic representation of the control means 3. It comprises a microcontroller 50 with integral analogue to digital converter, memory, control and display driver circuits. Power for the microcontroller is provided by the battery power pack 51 and a clock signal is provided by the crystal oscillator 52. A low power ZigBee module 53 provides wireless connectivity to the data logger 11 via the USB RF transceiver 12 for short-range communication. A custom data exchange protocol enables the reliable transfer of pressure and temperature measurements to the remote logger. Security is enabled to prevent eaves dropping as well as providing for message authentication and integrity checking on the received sensor measurements. Data is streamed in real-time to the remote data logger. Received data is displayed in real-time on a visual display to the user. A control interface allows the user to configure display options for one or more channels of received data. The received data is stored for offline retrieval in the remote logger or alternatively may be stored in the SD card 55. Real-time analysis can be performed permitting feature extraction and data fusion.

Calibration checking of the apparatus may be easily and conveniently performed using dead-weights specially adapted to facilitate easy placement onto the sensor port area of the transducer. The overall pressure measurement sequence may be completed within 1 ms thereby providing a dynamic signal measurement rate of 1 kHz. Interface pressure readings may be stored, within the microcontroller memory or in an SD card if prolonged data logging is desired, for later recall and display or alternatively may be streamed to a computer or data logger 11 via the USB wireless transceiver 12 for further analysis, manipulation and display.

It will be clear to those skilled in the art that alternative sensor signal chain designs may be used to provide estimates of the tongue applied pressure. For instance, a MEMS pressure sensor such as those of the MS58 series manufactured by Measurement Specialities Inc., Switzerland may be used in the modified MEMS pressure sensor of this invention. This series includes integrated ADC, 64 BIT PROM memory, and digital interface (I2C and serial SPI interface) integrated circuits on the MEMS pressure sensor chip and come with factory determined calibration and temperature compensation coefficients stored in PROM memory. A microcontroller may be used to provide software adjustment of the digital signal available via standard three wire serial interface in order to provide dynamic temperature compensation for offset and span using the stored factory determined calibration coefficients. The digital interface is particularly useful in multisensory embodiments of the present invention since specific sensors may be individually addressed on a common interface connection.

Similarly, it will be clear to those versed in the art that the flexible substrate and cable may incorporate electrical screening to reduce RF noise interference in noisy environments.

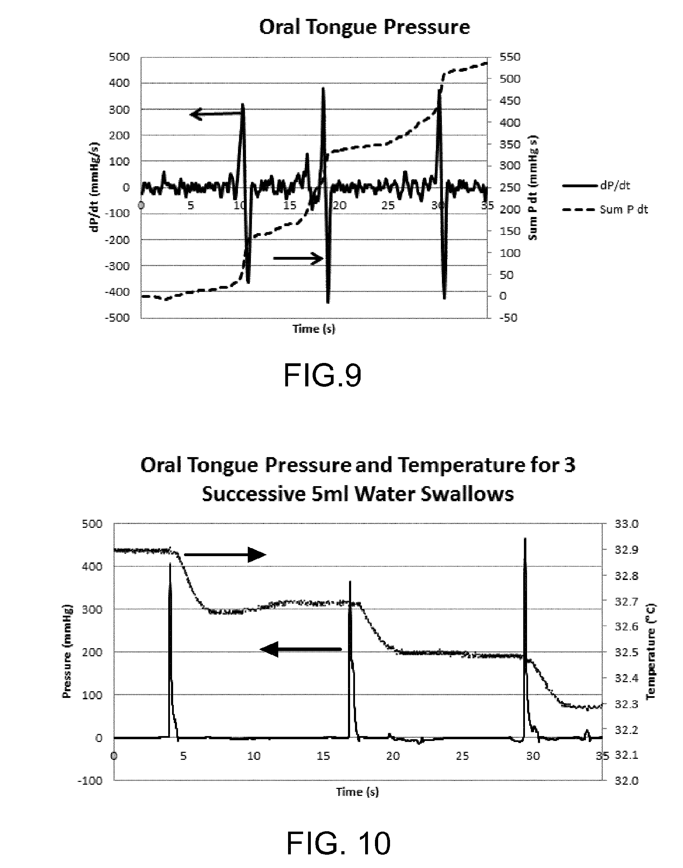

The functionality of the present invention will be better understood by reference to Figs 8 and 9. Fig. 8 contains a plot of data obtained using the intraoral interface pressure measurement system of this invention with intraoral interface pressure transducer placed between the upper front teeth and the alveolar ridge. The data demonstrates the responsiveness of the system to changes in tongue pressure and in particular demonstrates the fast response times of the system and its associated capabilities for tracking fast changing pressure waves. Fig. 9 shows pressure gradient and pressure impulse data algorithmically derived from the pulse data of Fig. 8.

As the transducers also measure the temperature in real time, it is possible to use such temperature data to distinguish between bolus swallow pressure pulses and normal non-bolus swallows provided that there is a temperature differential between bolus temperature and body temperature. This marker function of the temperature trace is illustrated in Fig. 10.

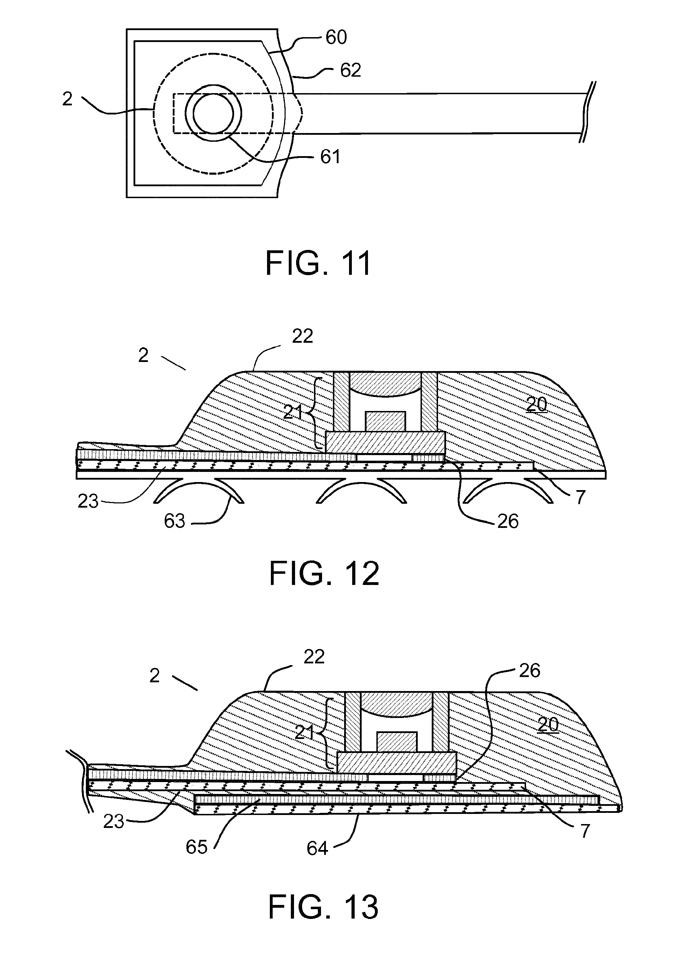

Fig. 11 is a schematic representation of one means of affixing the transducer to a supporting tissue using medically approved adhesive tape 60. The tape is prepunched with a circular hole 61 which has a larger diameter than the sensor port which may be concentrically located over the senor port to allow un-impeded access to the port by the tongue or other pressure applying tissue. The overall size and shape of the adhesive tape is such as to extend beyond the footprint of the sensor thereby providing an adhesive border to hold the transducer in place on the supporting tissue. A release liner 62 is provided to protect the adhesive tape prior to deployment. The release liner also facilitates sensor deployment using surgical gloves.

Fig 12 is a schematic representation of yet another embodiment of the transducer. In this embodiment, integral mini suction cups 63 are provided to allow device adhesion to supporting tissue without the need for fixative pastes, tabs or tape.

Fig. 13 is a schematic representation of yet another embodiment of the transducer whereby an adhesion promotion layer 64 is bonded to the bottom surface 23 of the transducer via an intermediate layer 65. The adhesion promotion layer 64 may be formed from a polar polymer film such as Dupont Kapton™ polyimide. A metallic film such as copper may be used as the intermediate layer 65 and is adhesively joined to the adhesion promotion layer 64 to form copper-clad polyimide. The metallic intermediate layer 65 is easily bonded to the bottom surface 23 of the transducer.

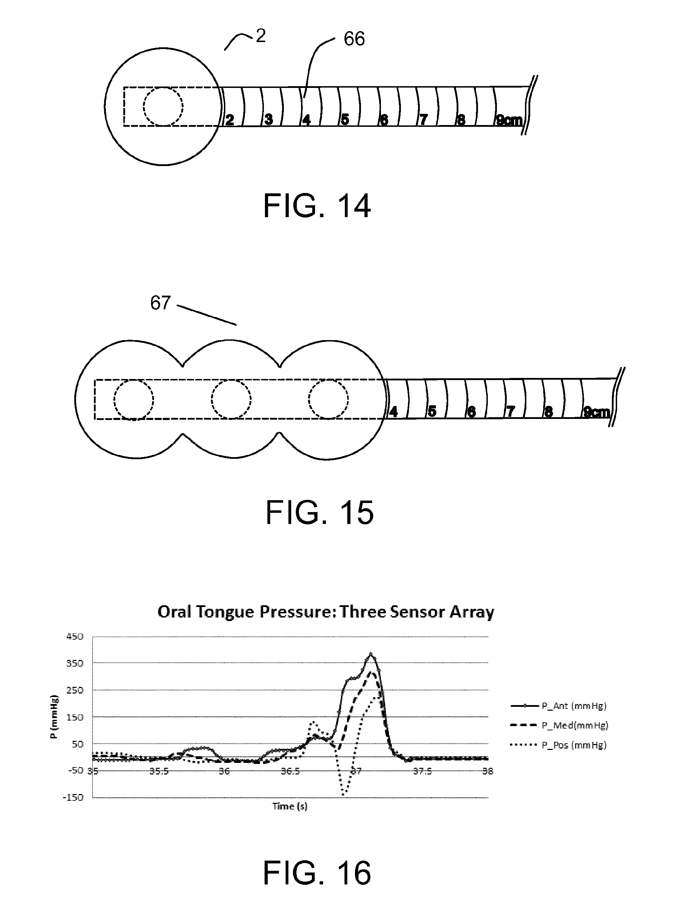

Fig. 14 shows the transducer of this invention with a graduated length scale 66 marked in suitable units such as centimeters. This scale provides the clinician with a convenient means of recording the exact position of the transducer relative to anatomical landmarks such as the patient's upper teeth and lips.

An array of modified MEMS pressure sensors could be integrated into a single conformable semisolid body 67 to provide an alternative embodiment designed according to the principles of this invention and illustrated in Fig. 15, which would provide pressure wave data at a plurality of locations within the oral cavity. The fast transducer response times allows the discrimination of peak pulse propogation times which in turn may be used to infer bolus velocity through its general correlation with pressure pulse velocity. This is illustrated in Fig. 16 where clear and measurable time delays are evident between pressure maxima for anterior, medial and posterior transducers.

Single use sleeves with elasticated opening, which could be slipped over the intraoral interface pressure transducer, formed from biomedically approved and compatible non-elastic thin flexible membranes, could be used in order to conform to healthcare hygiene and patient comfort and safety guidelines.

Similarly, it will be clear to those skilled in the art that the shape of the conformable semisolid body may be chosen from a wide range of geometric shapes including ellipsoid, square, rectangular and other to suit specific biomedical measurement applications.

The oral manometer of this invention may be sterilized by heating in an autoclave or by exposure to sterilization gas or using commercial wipe systems.

The invention is not limited to the embodiment hereinbefore described which may be varied in construction and detail without departing from the spirit of the invention.

## Claims

1. A method of measuring intraoral pressure at a tissue interface within the oral cavity of a person, which method employs an intraoral interface pressure transducer apparatus (1) comprising a pressure transducer (2) and control means (3) in communication with the pressure transducer and configured to provide an interfacing, storage or display function, the pressure transducer comprising:
- a conformable semi-solid body (20) that in use is capable of at least partially conforming to the shape of a tissue surface in the oral cavity;
- a modified MEMS pressure sensor (21) contained within the conformable semi-solid body (20), the modified MEMS pressure sensor having a pressure sensor port (28) adapted to allow direct contact to continuum materials and configured to generate a pressure signal indicative of interface pressure applied to the tissue within the oral cavity,
the method comprising the steps of locating the intraoral interface pressure transducer (2) in the oral cavity of the person at a tissue interface, wherein the modified MEMS pressure sensor (21) produces a pressure signal indicative of the intraoral pressure at the tissue interface which is communicated to the control means (3).

2. A method as claimed in Claim 1 in which the pressure sensor port (28) of the modified MEMS pressure sensor (21) has an internal volume that is partially filled with a bottom layer of MEMS protection material (29) and a top layer of media isolation material (44) such that a top surface (31) of the media isolation material is substantially co-planar with a top surface (32) of the pressure sensor port (28).

3. A method as claimed in Claim 1 in which the control means (2) is in electrical communication with the modified MEMS pressure sensor (21) by means of a patterned conductor (26) on a flexible substrate (7).

4. A method as claimed in any preceding Claim in which the apparatus comprises a head fixture (8) adapted to attach to the head of the person, support the control means (3), and anchor the pressure transducer (2).

5. A method as claimed in any preceding Claim, in which the apparatus further includes a remote data logger (11) with wireless transceiver (12), wherein the control means (3) comprises means for providing wireless connectivity with the data logger, wherein the method comprises an additional step of wirelessly communicating the pressure signal from the control means to the data logger.

6. An intraoral interface pressure transducer apparatus (1) comprising a pressure transducer (2) and control means (3) in communication with the pressure transducer and configured to provide interfacing, storage, or display function, the pressure transducer comprising:
- a conformable semi-solid body (20) that in use is capable of at least partially conforming to the shape of a tissue surface in the oral cavity;
- a modified MEMS pressure sensor (21) contained within the conformable semi-solid body (20), the modified MEMS pressure sensor having a pressure sensor port (28) adapted to allow direct contact to continuum materials and configured to generate a pressure signal indicative of interface pressure applied to the tissue within the oral cavity; and
- means for affixing the conformable semi-solid body to a surface of the oral cavity.

7. An intraoral interface pressure transducer apparatus as claimed in Claim 6 in which the affixing means is selected from a fixative cream or paste, an adhesive tape or film, or a suction cup.

8. An intraoral interface pressure transducer apparatus as claimed in Claim 6 or 7 and further including a head fixture (8) adapted to attach to the head of a user and support the control means (3) and anchor the pressure transducer (2).

9. An intraoral interface pressure transducer apparatus as claimed in Claim 6, 7 or 8 in which the head fixture (8) comprises a head set adapted to attach to the head, and an adjustable boom (10) that is mounted to the head set for pivotal movement relative to the head set, and wherein the pressure transducer (2) is anchored to a lock interconnect (4) at the free end of the adjustable boom.

10. An intraoral interface pressure transducer apparatus as claimed in any of Claims 6 to 9 in which the control means (3) is configured to provide wireless connectivity with a remote data logger (11).

11. An intraoral interface pressure transducer apparatus as claimed in any of Claims 6 to 10 in which the pressure sensor port (28) of the modified MEMS pressure sensor (21) has an internal volume that is partially filled with a bottom layer of MEMS protection material (29) and a top layer of media isolation material (44) such that a top surface (31) of the media isolation material (30) is substantially co-planar with a top surface (32) of the pressure sensor port (28).

12. An intraoral interface pressure transducer apparatus (1) as claimed in any of Claims 6 to 10 further comprising a head-fixture (8) adapted to support the control means (3).

13. An intraoral interface pressure transducer apparatus (1) according to any of Claims 10 to 11 in which: the MEMS protection material is a low viscosity low hardness incompressible gel; and/or in which the MEMS media isolation material is a soft, non-stick, incompressible rubber or gel.

14. A kit comprising an intraoral interface pressure transducer apparatus (1) according to any of Claims 6 to 12, and a data logger (11) having a wireless transceiver (12), wherein the control means (3) comprises means for providing wireless connectivity with the data logger (11).

15. A kit comprising an intraoral interface pressure transducer apparatus (1) according to any of Claims 6 to 12, and a computer program stored on a storage medium, the computer program comprising program instructions adapted to process pressure data received from the pressure transducer.
